(19) Europäisches Patentamt
European Patent Office
Office européen des brevets

(11) **EP 3 988 031 A1**

(12) **EUROPEAN PATENT APPLICATION**
published in accordance with Art. 153(4) EPC

(43) Date of publication:
**27.04.2022 Bulletin 2022/17**

(21) Application number: **20827410.0**

(22) Date of filing: **11.06.2020**

(51) International Patent Classification (IPC):
*A61B 10/00* (2006.01)     *A61B 5/0285* (2006.01)

(52) Cooperative Patent Classification (CPC):
**A61B 5/0285; A61B 10/00**

(86) International application number:
**PCT/JP2020/022939**

(87) International publication number:
**WO 2020/255840 (24.12.2020 Gazette 2020/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.06.2019 JP 2019114591**

(71) Applicant: **Sony Group Corporation
Tokyo 108-0075 (JP)**

(72) Inventor: **OKUBO, Atsushi
Tokyo 108-0075 (JP)**

(74) Representative: **MFG Patentanwälte
Meyer-Wildhagen Meggle-Freund
Gerhard PartG mbB
Amalienstraße 62
80799 München (DE)**

(54) **INFORMATION PROCESSING DEVICE, INFORMATION PROCESSING METHOD, AND PROGRAM**

(57)     The information processing apparatus according to the present technology includes a computation unit. The computation unit computes an evaluation value related to heart disease on the basis of a detection result of a blood flow rate. Accordingly, the heart disease status can be easily ascertained. For example, the BNP level and the NT-pro BNP level can be estimated non-invasively and bloodlessly without requiring a blood test at a medical institution. Moreover, remote monitoring at home and the like becomes possible and telemedicine systems of heart disease can be built, for example, because the measurement at a medical institution is unnecessary.

FIG.1

EP 3 988 031 A1

# Description

## Technical Field

The present technology relates to an information processing apparatus, an information processing method, and a program that can be applied to diagnosis of heart disease and the like.

## Background Art

Various types of blood flow velocity sensors for measuring the velocity of a blood flow through a blood vessel have been developed. For example, Patent Literature 1 has disclosed a laser Doppler flowmeter.

The laser Doppler flowmeter irradiates a living body with laser light. The laser light is reflected by biological tissues and becomes scattered light. Scattered light reflected by particles moving in a blood flow, such as red blood cells, undergoes a wavelength shift (Doppler shift) with respect to scattered light reflected by other immobile tissues, the wavelength shift (Doppler shift) being caused by the Doppler effect.

Detecting scattered light and obtaining a signal change involved with the Doppler shift can measure movement velocity, i.e., blood flow velocity of particles in blood, such as red blood cells. Moreover, the use of this measurement principle can also measure velocity of a group of particles moving through an object to be measured, other than particles in blood.

## Citation List

## Patent Literature

Patent Literature 1: Japanese Patent Application Laid-open No. 2017-192629

## Disclosure of Invention

## Technical Problem

In recent years, a BNP or NT-pro BNP level in blood have been widely used for diagnosis and the like as biomarkers indicating a heart disease status. The measurement of the BNP or NT-pro BNP level requires a blood test. Therefore, it is necessary to regularly visit a medical institution, for example.

In view of the above-mentioned circumstances, it is an object of the present technology to provide an information processing apparatus, an information processing method, and a program that can easily ascertain a heart disease status.

## Solution to Problem

In order to accomplish the above-mentioned object, an information processing apparatus according to the present technology includes a computation unit.

The computation unit computes an evaluation value related to heart disease on the basis of a detection result of a blood flow rate.

In this information processing apparatus, the evaluation value related to heart disease is computed on the basis of the detection result of the blood flow rate. Accordingly, the heart disease status can be easily ascertained.

The computation unit may compute the evaluation value in accordance with a predetermined learning algorithm.

The computation unit may compute, as the evaluation value, at least one of a brain natriuretic peptide level or an N-terminal prohormone of brain natriuretic peptide level.

The detection result of the blood flow rate may include a detection result obtained by a laser Doppler flowmeter.

The computation unit may divide a blood flow velocity into a plurality of velocity sections and compute the evaluation value on the basis of a blood flow rate in each velocity section.

The computation unit may compute the evaluation value on the basis of a ratio of the blood flow rate in each velocity section.

The computation unit may divide a blood flow velocity into a plurality of velocity sections and compute the evaluation value on the basis of a blood flow rate in a predetermined velocity section.

The computation unit may compute the evaluation value on the basis of a ratio of the blood flow rate in the predetermined velocity section to a blood flow rate in all the velocity sections.

The predetermined velocity section may be a low-velocity-side velocity section of the plurality of velocity sections.

The computation unit may compute the evaluation value on the basis of a time-series variation of the detection result of the blood flow rate.

The information processing apparatus may further include a detection device capable of detecting the blood flow rate.

The detection device may be a laser Doppler flowmeter.

The information processing apparatus may further include a generation unit that generates notification information related to heart disease on the basis of the computed evaluation value.

The information processing apparatus may further include a notification unit that notifies a user of the generated notification information.

The information processing apparatus may be configured as a wearable device or a portable terminal.

The information processing apparatus may be configured as a vehicle-mounted device or a part of a robot.

An information processing method according to an embodiment of the present technology is an information processing method executed by a computer system and includes computing an evaluation value related to heart disease on the basis of a detection result of a blood flow rate.

A program according to an embodiment of the present technology causes a computer system to execute the following step.

A step of computing an evaluation value related to heart disease on the basis of a detection result of a blood flow rate.

Brief Description of Drawings

[Fig. 1] A schematic diagram showing the outline of a heart-disease check system according to an embodiment.
[Fig. 2] A flowchart showing an operation example of the heart-disease check system.
[Fig. 3] A schematic diagram for describing a Doppler shift and a Doppler beat.
[Fig. 4] A schematic diagram showing a principal configuration example of a laser Doppler flowmeter.
[Fig. 5] A schematic diagram showing a Doppler shift caused by movements of scatterers.
[Fig. 6] A schematic diagram showing a specific configuration example of the laser Doppler flowmeter.
[Fig. 7] An example of a beat signal output from a light-receiving portion.
[Fig. 8] An example of a signal processing result of a beat signal output from the light-receiving portion.
[Fig. 9] A schematic diagram for describing a generation example of a learned model for computing a BNP level.
[Fig. 10] A diagram for describing computation of a heart-disease evaluation value based on blood flow velocity sections.
[Fig. 11] A flowchart showing a computation example of the heart-disease evaluation value based on the blood flow velocity sections.
[Fig. 12] A schematic diagram showing a configuration example of a heart-disease check apparatus according to the embodiment.
[Fig. 13] A block diagram showing a hardware configuration example of an evaluation value computation device.

Mode(s) for Carrying Out the Invention

[Heart-Disease Check System]

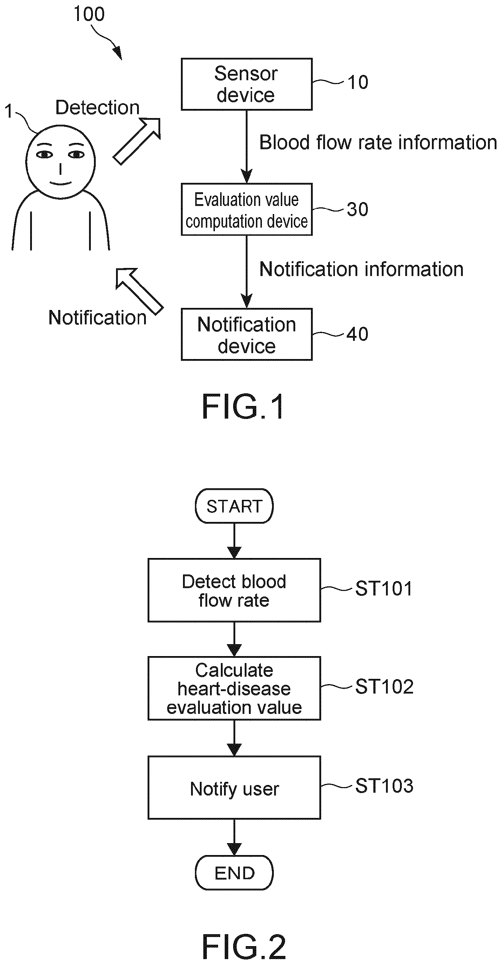

Fig. 1 is a schematic diagram showing the outline of a heart-disease check system according to an embodiment of the present technology. Fig. 2 is a flowchart showing an operation example of the heart-disease check system.

The heart-disease check system corresponds to an embodiment of an information processing system according to the present technology. It should be noted that the heart-disease check can also be referred to as simple heart-disease diagnosis.

A heart-disease check system 100 includes a sensor device 10, an evaluation value computation device 30, and a notification device 40.

The sensor device 10, the evaluation value computation device 30, and the notification device 40 are communicably connected with each other. The connection form between the respective devices is not limited, and the respective devices are configured to be capable of network communication or near-field communication with a wire or wirelessly. Alternatively, the respective devices may be directly connected via cables or the like.

The sensor device 10 detects a blood flow rate of a user 1 who utilizes the heart-disease check system 100 (Step 001).

The specific configuration of the sensor device 10 is not limited, and an arbitrary configuration capable of detecting the blood flow rate of the user 1 may be employed. Moreover, a method (e.g., algorithm) and the like for detecting the blood flow rate of the user 1 are also not limited, and an arbitrary technology may be used.

The sensor device 10 generates blood flow rate information as a detection result of the blood flow rate. The sensor device 10 then outputs the blood flow rate information to the evaluation value computation device 30. It should be noted that the blood flow rate information includes arbitrary information related to the blood flow rate.

In this embodiment, the sensor device 10 functions as a detection device capable of detecting the blood flow rate.

Based on the blood flow rate information output from the sensor device 10, the evaluation value computation device 30 computes a heart-disease evaluation value (Step 102).

The heart-disease evaluation value is an evaluation value related to heart disease and includes an evaluation value related to an arbitrary disease (symptom) included in the heart disease. For example, the heart-disease evaluation value includes evaluation values related to heart failure, angina pectoris, myocardial infarction, aortic aneurysm, aortic dissection, valvular heart disease, cardiomyopathy, atrial septal defect, arrhythmia, and the like.

Examples of the criteria for evaluation can include risk of heart disease, a degree of progress of heart disease, a severity level of heart disease, and the like. The present technology is not limited thereto, and the criteria for evaluation includes an arbitrary evaluation criteria related to heart disease.

A method of computing the heart-disease evaluation value is not limited, and an arbitrary technology (algorithm or the like) may be used. For example, an arbitrary machine learning algorithm using a deep neural network (DNN) or the like may be used. For example, the use of artificial intelligence (AI) or the like that performs deep learning can improve the accuracy of computation of the heart-disease evaluation value.

For example, the evaluation value computation device 30 includes a learning unit and an identification unit (not shown). The learning unit performs machine learning on the basis of input information (learning data) and outputs the learning result. Moreover, the identification unit performs identification (determination, prediction, and the like) of the input information on the basis of the input information and the learning result.

For example, a neural network and deep learning are used for the learning method in the learning unit. The neural network is a model that mimics neural networks of a human brain. The neural network is constituted by three types of layers of an input layer, an intermediate layer (hidden layer), and an output layer.

Deep learning is a model using neural networks with a multi-layer structure. Deep learning can repeat characteristic learning in each layer and learn complicated patterns hidden in mass data.

Deep learning is used for the purpose of identifying objects in an image or words in a speech. As a matter of course, deep learning can also be applied to the computation of the heart-disease evaluation value according to this embodiment.

Moreover, a neuro chip/neuromorphic chip having the neural network concept can be used as a hardware structure for realizing such machine learning.

Moreover, supervised learning, unsupervised learning, semi-supervised learning, reinforcement learning, inverse reinforcement learning, active learning, transfer learning, and the like exist for problem settings in machine learning.

For example, supervised learning learns feature amounts on the basis of provided labeled learning data (training data). Accordingly, labels of unknown data can be derived.

Moreover, unsupervised learning analyzes a large amount of unlabeled learning data, extracts feature amounts, and performs clustering on the basis of the extracted feature amounts. Accordingly, trend analysis and future prediction can be performed on the basis of a huge amount of unknown data.

Moreover, semi-supervised learning is mixed supervised learning and unsupervised learning. The semi-supervised learning is a method in which feature amounts are learned in supervised learning, and then a large amount of training data is provided in unsupervised learning and learning is repeatedly performed while feature amounts are automatically computed.

Moreover, reinforcement learning handles a problem in that an agent in a certain environment observes a current state and determines an action that the agent should take. The agent selects an action to thereby get a reward from the environment and learns a policy that can maximize the reward through a series of actions.

In this manner, learning an optimal solution in a certain environment can reproduce the human judgement ability and can also cause a computer to learn a judgement ability beyond the human judgement ability.

The evaluation value computation device 30 is also capable of generating virtual sensing data by machine learning. For example, the evaluation value computation device 30 is capable of predicting from certain sensing data other sensing data and using the other sensing data as input information, e.g., generating position information from input image information.

Moreover, the evaluation value computation device 30 is also capable of generating from a plurality of pieces of sensing data other sensing data. Moreover, the evaluation value computation device 30 is also capable of predicting necessary information and generating predetermined information from sensing data.

Moreover, an arbitrary learning algorithm and the like different from those of machine learning may be used. Calculating an evaluation value in accordance with a predetermined learning algorithm can improve the accuracy of computation of the evaluation value. As a matter of course, the present technology is not limited to the case where the learning algorithm is used.

It should be noted that the learning algorithm may be applied to arbitrary processing in the present disclosure.

The evaluation value computation device 30 generates notification information related to heart disease on the basis of the computed heart-disease evaluation value. The evaluation value computation device 30 then outputs the notification information related to heart disease to the notification device 40. The notification information includes arbitrary information for notifying the user 1 of the evaluation related to heart disease. Video information, audio information, or the like that expresses, for example, "you have risk for heart failure", "your symptom is severe, so you should go to a hospital", or the like is generated as the notification information.

It should be noted that the computed heart-disease evaluation value itself is also included in heart-disease evaluation information.

The notification device 40 notifies the user 1 of the notification information output from the evaluation value computation device 30 (Step 103).

For example, the notification device 40 includes a display, a speaker, and the like and notifies the user 1 of the notification information through a moving image or a speech.

It should be noted that the user 1 may be notified by tactile presentation through a vibration function or the like. In this case, the evaluation value computation device 30 generates information (e.g., information or the like for generating a vibration or the like) for presenting a predetermined tactile sense based on the heart-disease evaluation value, as the notification information. The evaluation value computation device 30 then outputs the information to the notification device 40. The notification device 40 presents the predetermined tactile sense based on the notification information to the user 1 through the vibration function or the like.

Moreover, the user 1 may be notified by emission of light having a predetermined color (including an emission pattern and the like). In this case, the evaluation value computation device 30 generates information for performing emission of light having a predetermined color based on the heart-disease evaluation value, as the notification information. The evaluation value computation device 30 then outputs the information to the notification device 40. The notification device 40 controls a light source or the like to thereby emit the light having the predetermined color based on the notification information.

In this manner, the information for notifying the user 1 is also included in the notification information. Moreover, driving a predetermined device or the like on the basis of the notification information to notify the user 1 is included in the notification of the notification information.

In addition, an arbitrary technology may be employed.

The sensor device 10, the evaluation value computation device 30, and the notification device 40 may be integrally configured as a single apparatus. Moreover, arbitrary two devices of those devices may be configured as a single apparatus.

[Principles of Laser Doppler Blood Flowmeter]

A laser Doppler flowmeter (LDF) can be used as the sensor device 10. The laser Doppler flowmeter is a measurement device utilizing a Doppler shift of light.

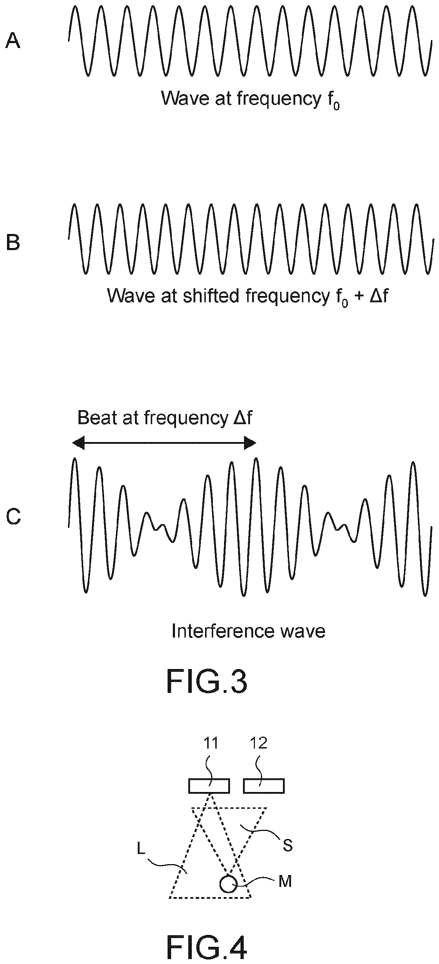

Fig. 3 is a schematic diagram for describing a Doppler shift and a Doppler beat.

It is assumed that a wave at a frequency fo arrives at a measurement point as shown Fig. 3A. It is also assumed that a wave at the same frequency fo is reflected by a mobile object and arrives at the same measurement point.

The wave reflected by the mobile object has a frequency shift due to the Doppler phenomenon and the frequency of the wave is $f_0 + \Delta f$ as shown in Fig. 3B.

It is known that an interference wave of the original wave shown in Fig. 1A and the Doppler-shifted wave shown in Fig. 1B beats due to the beat phenomenon at the frequency $\Delta f$. The wave at the frequency $\Delta f$ will be referred to as a Doppler beat and the frequency $\Delta f$ will be referred to as a beat frequency.

Measuring the frequency $\Delta f$ (beat frequency) of the Doppler beat can determine velocity of the mobile object causing the Doppler shift with respect to the measurement point without measuring the frequency $fo + \Delta f$ of the wave having the Doppler shift.

Fig. 4 is a schematic diagram showing a principal configuration example of the laser Doppler flowmeter.

As shown in Fig. 4, a light-emitting portion 11 emits laser light L. When the laser light L enters a scatterer M, the scatterer M reflects the laser light L as scattered light S. The scattered light S enters a light-receiving portion 12 and is detected.

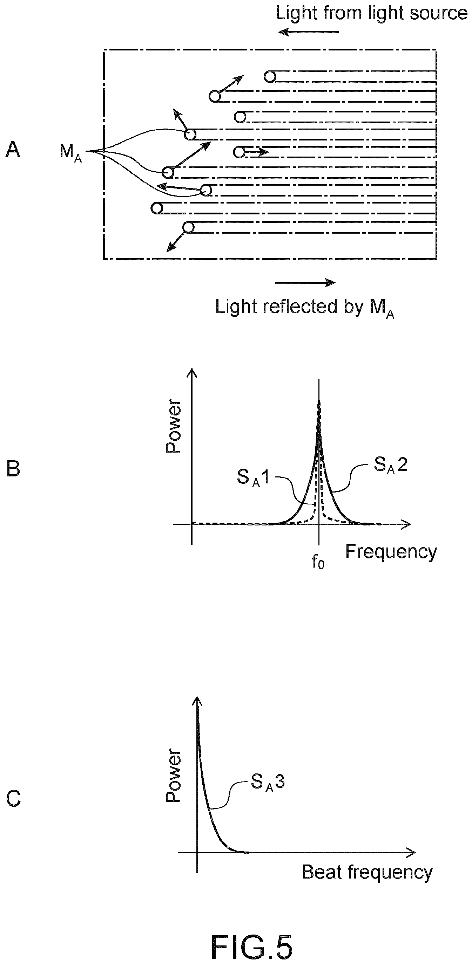

Fig. 5 is a schematic diagram showing a Doppler shift caused by movements of scatterers.

As shown in Fig. 5, it is assumed that a large number of scatterers MA are moving in various directions in light emitted from a light source. In the figure, the arrows of the scatterers MA denote velocity vectors of the scatterers MA. The scatterers MA without the arrows indicate that these scatterers MA are stationary.

Fig. 5B shows a power spectrum SA1 of scattered light associated with the stationary scatterers MA and a power spectrum SA2 of scattered light associated with the moving scatterers MA.

As to a blood flow measurement example, the stationary scatterers $M_A$ are body tissues and the like and the moving scatterers MA are red blood cells and the like.

As shown in Fig. 5B, the movement velocity of the respective scatterers MA with respect to the light source is different, and therefore the power spectrum SA2 of the scattered light is a combined wave of Doppler shifts caused by a large number of scatterers MA.

In a case where an amount ratio of the stationary scatterers MA is much higher than an amount ratio of the moving scatterers MA, the scattered light of the stationary scatterers MA has intensity much higher than the scattered light of the moving scatterers MA. Thus, most interference between scattered light that causes Doppler beats are interference between the scattered light of the stationary scatterers MA and the scattered light of the moving scatterers MA.

Fig. 5C shows a power spectrum SA3 of the Doppler beat caused by interference between scattered light SA1 and scattered light SA2. The frequency of SA3 is an absolute value of a difference between the frequency of SA1 and the frequency of SA2.

The shape of this power spectrum SA3 represents a velocity distribution of the scatterers MA with respect to the light source. Therefore, considering a decrease of a Doppler shift amount that depends on movement directions of the scatterers MA, an actual velocity distribution of the scatterers $M_A$ can be determined.

It should be noted that the technology that measures a Doppler shift caused by a large number of such objects to be observed, which are moving in respective directions, are called dynamic light scattering (DLS).

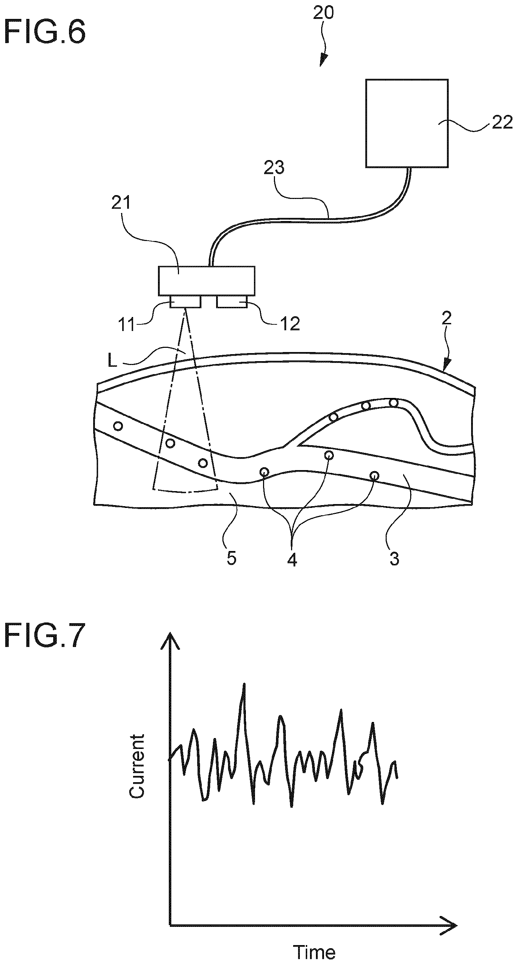

Fig. 6 is a schematic diagram showing a specific configuration example of laser Doppler measurement.

A laser Doppler flowmeter 20 (hereinafter, referred to as an LDF) includes a sensor head 21 and a signal processing device 22 that are communicably connected with each other.

In Fig. 6, the sensor head 21 and the signal processing device 22 are connected to each other via a signal line 23. The connection form between both devices is not limited, and wireless communication or the like may be used.

The sensor head 21 includes a light-emitting portion 11 that emits laser light L and the light-receiving portion 12 that receives scattered light. The light-emitting portion 11 is, for example, a laser light source. The light-receiving portion 12 is, for example, a photodiode.

The sensor head 21 is disposed in proximity to or in close-contact with a living body 2 that is an object to be measured. The living body 2 has red blood cells 4 flowing through a blood vessel 3 and a stationary tissue 5. The stationary tissue 5 is a stationary biological tissue other than the blood.

The LDF 20 irradiates the living body 2 with the laser light L from the light-emitting portion 11. The laser light L is scattered by the red blood cells 4 and the stationary tissue 5, received by the light-receiving portion 12, and converted into an electrical signal.

Fig. 7 is an example of the electrical signal output from the light-receiving portion 12.

The scattered light that enters the light-receiving portion 12 includes light scattered by the red blood cells 4 moving through the blood flow and light scattered by the stationary tissue 5 not moving.

Therefore, the signal shown in Fig. 7 are a set of many Doppler beats caused by interference of both scattered light. In the present disclosure, the signal including such a set of Doppler beats will be referred to as a beat signal.

The signal processing device 22 acquires the beat signal from the sensor head 21 via the signal line 23. Moreover, the signal processing device 22 performs signal processing, such as Fourier transform, on the received beat signal.

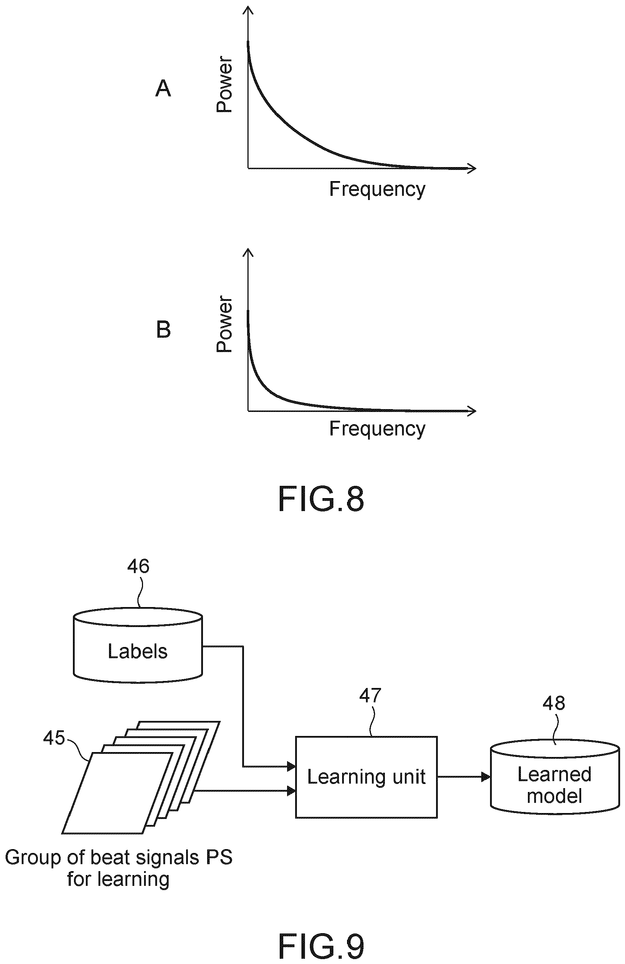

Fig. 8 is an example of a signal processing result.

Fig. 8A is a graph in which a beat signal measured at a fingertip is converted in a frequency band. Fig. 8B is a graph in which a beat signal measured at a wrist is converted in a frequency band.

The power spectra shown in those graphs represent the power with respect to each beat frequency and corresponds to distribution density of red blood cells moving at velocity corresponding to the beat frequency. Moreover, the shape of the power spectrum corresponds to a velocity distribution of red blood cells with respect to the light-emitting portion 11.

For example, components having high beat frequency are components corresponding to red blood cells moving at high velocity, of red blood cells flowing through the blood vessel, and corresponds to high-velocity components having high blood flow velocity in the blood flowing through the blood vessel 3. Components having low beat frequency are components corresponding to red blood cells moving at low velocity, of red blood cells flowing through the blood vessel, and corresponds to low-velocity components having low blood flow velocity in the blood flowing through the blood vessel 3.

In Fig. 8A, a power attenuation rate with respect to an increase in beat frequency is lower as compared to Fig. 8B. As a result, it can be seen that the rate of red blood cells having high movement velocity is higher at the fingertip than at the wrist.

Moreover, since the total area of the power spectrum in Fig. 8A is wider than that in Fig. 8B, it can be seen that red blood cells moving in the observation region are larger in number at the fingertip.

The signal processing device 22 is capable of computing, on the basis of a power spectrum of a beat signal (hereinafter, referred to as a beat signal PS), blood-flow mean velocity, a blood flow rate, and the like. Moreover, the signal processing device 22 is also capable of computing, on the basis of a time-series variation of the beat signal PS, high-order blood flow-related information such as changes (pulses) of the blood-flow mean velocity over time, pulse rate, and blood flow velocity.

As described above, the blood flow rate information that is the detection result of the blood flow rate includes the arbitrary information related to the blood flow rate. For example, an arbitrary detection result obtained by the LDF 20 is included in the blood flow rate information.

That is, the beat signal PS and the time-series variation of the beat signal PS, which are shown in Fig. 8 and the like, are included in the blood flow rate information. Moreover, arbitrary information computed on the basis of the beat signal PS and the time-series variation of the beat signal PS is also included in the blood flow rate information.

[Evaluation Value Computation Device]

The evaluation value computation device 30 functions as an embodiment of an information processing apparatus according to the present technology.

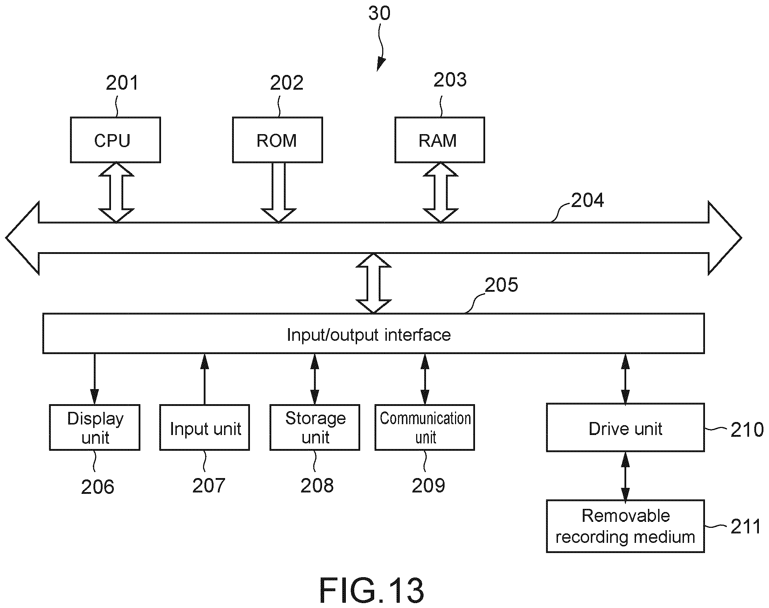

The evaluation value computation device 30 has, for example, hardware necessary for computer configurations such as a CPU, a ROM, a RAM, and an HDD (see Fig. 13). An information processing method according to the present technology is executed by, for example, the CPU loading a program according to the present technology, which is recorded in the ROM or the like in advance, into the RAM and executing the program according to the present technology.

For example, an arbitrary computer such as a personal computer (PC) can realize the evaluation value computation device 30. As a matter of course, hardware such as a GPU, an FPGA, and an ASIC may be used.

In this embodiment, the computation unit and the generation unit serving as the functional blocks are configured when the CPU executes a predetermined program. As a matter of course, dedicated hardware such as an integrated circuit (IC) may be used for realizing the functional blocks.

The program is, for example, installed in the evaluation value computation device 30 via various recording media. Alternatively, the program may be installed via the Internet or the like.

The type and the like of the recording medium on which the program is recorded are not limited, and an arbitrary computer-readable recording medium may be used. For example, an arbitrary computer-readable non-transitory storage medium may be used.

In this embodiment, a brain natriuretic peptide (BNP) level is computed as the heart-disease evaluation value. The BNP in the blood is a hormone secreted from ventricles when stress is applied on the heart. As BNP concentration becomes higher (the BNP level is higher), the risk that the action of the heart may be weakened becomes higher.

For example, the following evaluation can be performed.

BNP levels of 0 to 20: Normal group (cardiovascular system has no problem)

BNP levels of 20 to 40: High-risk group (heart failure is unlikely)

BNP levels of 40 to 100: Asymptomatic group 1 (heart failure is possible)

BNP levels of 100 to 200: Asymptomatic group 2 (heart failure is likely)

BNP levels of 200 or more: Symptomatic group 1 (heart failure is highly likely)

In this manner, the BNP level is used as a parameter for evaluating the risk (level) for heart failure. It should be noted that the evaluation based on the BNP level is not limited to the classification described above.

For example, when the erythrocyte deformability lowers, the flow of erythrocytes (red blood cells) moving through a capillary vessel slow down and the blood flow rate of the capillary vessel lowers. When the blood flow rate of the capillary vessel lowers, oxygen supply is reduced, which leads to deterioration of the cardiac function. As a result, the load (stress) on the heart increases and the BNP level increases.

For example, it is known that healthy subjects and patients suffering from heart disease can also be distinguished by measuring a blood-flow velocity ratio or an occupancy rate of red blood cells hard to flow.

Computing the BNP level as the heart-disease evaluation value on the basis of the blood flow rate information detected by the sensor device 10 was newly found on the basis of such knowledge. For example, the BNP level can be estimated on the basis of the beat signal PS detected by the LDF 20.

Fig. 9 is a schematic diagram for describing a generation example of a learned model for computing the BNP level.

A group 45 of beat signals PS for learning and labels 46 are input into a learning unit 47.

The labels 46 are information associated with the beat signals PS for learning and are BNP levels. In this embodiment, the BNP levels are measured by conducting blood tests with respect to people from whom the beat signals PS were measured. The measured BNP levels are set as the labels 46 to the beat signals PS. Data set for learning is thus generated.

The data set for learning is, for example, manually generated. Alternatively, data set for learning generated in advance may be acquired and may be input into the learning unit 47.

The learning unit 47 performs learning using the data set for learning on the basis of a machine learning algorithm. Learning updates parameters (coefficients) for computing BNP levels, as learned parameters. A program in which the generated learned parameters are incorporated is generated as a learned model 48.

Through the learned model 48, a BNP level is computed with respect to an input beat signal PS.

As also illustrated in the above evaluation classification, blood tests can measure BNP levels at high accuracy. The BNP levels can be used as continuous quantitative values relating to a degree of progress of the symptom of the heart disease. Thus, high-accuracy data set for learning can be generated. As a result, a BNP level can be computed on the basis of a beat signal PS at very high accuracy.

An NT-pro BNP level can also be computed as the heart-disease evaluation value. As described below, the risk (level) for heart failure can be evaluated on the basis of the NT-pro BNP level.

NT-pro BNP levels of 0 to 55: Normal group (cardiovascular system has no problem)

NT-pro BNP levels of 55 to 125: High-risk group (heart failure is unlikely)

NT-pro BNP levels of 125 to 400: Asymptomatic group 1 (heart failure is possible)

NT-pro BNP levels of 400 to 900: Asymptomatic group 2 (heart failure is likely)

NT-pro BNP levels of 900 or more: Symptomatic group 1 (heart failure is highly likely)

It should be noted that the evaluation based on the NT-pro BNP level is not limited to such classification.

Like the BNP level, blood tests can measure NT-pro BNP levels at high accuracy. Thus, setting the measured NT-pro BNP levels as the labels 46 can built a high-accuracy learned model 48.

It should be noted that the estimation of the BNP level (NT-pro BNP level) according to the machine learning algorithm is not limited to the case where the beat signal PS is input. The BNP level (NT-pro BNP level) may be estimated by using, as the input, a time-series variation of the beat signal PS or arbitrary blood flow rate information generated on the basis of the beat signal PS (the time-series variation of the beat signal PS), such as blood flow velocity and a blood flow rate.

For example, data set for learning is generated by using blood flow rate information used as the input and a BNP level (NT-pro BNP level) computed by a blood test or the like, and a learned model 48 is generated. Accordingly, the BNP level (NT-pro BNP level) can be computed on the basis of the blood flow rate information at high accuracy.

[Blood Flow Velocity Section]

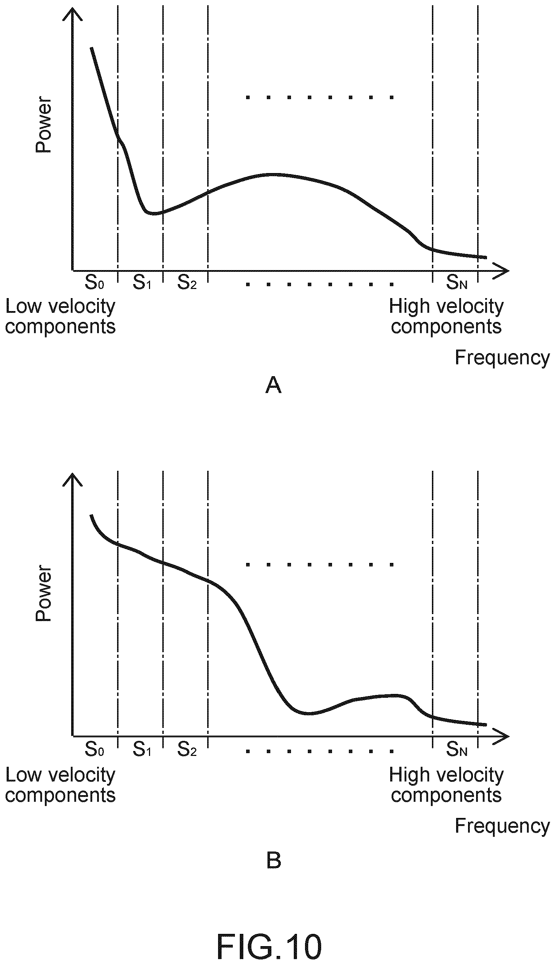

Fig. 10 is a diagram for describing computation of the heart-disease evaluation value based on blood flow velocity sections with respect to the beat signal PS.

As also described above, the beat signal PS corresponds to the velocity distribution of red blood cells (scatterers) in blood. The beat frequency on the horizontal axis corresponds to the red blood cell velocity. The vertical axis corresponds to the amount (number) of red blood cells moving at the velocity and corresponds to the blood flow rate of blood flowing at the velocity.

The high-frequency components of the beat frequency are high-velocity blood flow velocity components. The low-frequency components of the beat frequency are low-velocity blood flow velocity components.

As illustrated in Fig. 10A and B, the blood flow velocity is divided into a plurality of velocity sections $S_1$ to SN. That is, the beat frequency of the beat signal PS on the horizontal axis, which corresponds to the red blood cell velocity, is divided into the plurality of velocity sections $S_1$ to $S_N$. The heart-disease evaluation value is computed on the basis of the plurality of velocity sections $S_1$ to SN that are the blood flow velocity sections with respect to the beat signal PS.

For example, the heart-disease evaluation value can be computed on the basis of the blood flow rate in each velocity section. Alternatively, the heart-disease evaluation value can be computed on the basis of a ratio of the blood flow rate in each velocity section. The ratio of the blood flow rate in each velocity section is a parameter corresponding to the blood-flow velocity ratio.

For example, a predetermined velocity section is selected from the plurality of velocity sections $S_1$ to SN and a heart-disease evaluation value can be computed on the basis of a blood flow rate in the predetermined velocity section. It should be noted that the predetermined velocity section is not limited to a single velocity section, and the predetermined velocity section also includes a plurality of velocity sections. Thus, the predetermined velocity section can also be referred to as one or more predetermined velocity sections.

In a case where the predetermined velocity section is the plurality of velocity sections, the blood flow rate in the predetermined velocity section is typically a sum of respective blood flow rates in the plurality of velocity sections. The present technology is not limited thereto.

The evaluation value may be computed on the basis of a ratio of the blood flow rate in the predetermined velocity section to the blood flow rate in all the velocity sections. It should be noted that the blood flow rate in all the velocity sections is typically a sum of respective blood flow rates in all the velocity sections. The present technology is not limited thereto.

For example, a low-velocity-side velocity section of the plurality of velocity sections $S_1$ to SN may be selected as the predetermined velocity section. A blood flow rate in the low-velocity-side velocity section or a ratio of the blood flow rate in the low-velocity-side velocity section to the blood flow rate in all the velocity sections can be referred to as a parameter corresponding to the occupancy rate of red blood cells hard to flow.

It should be noted that the low-velocity-side velocity section is a velocity section on a lower velocity side as compared to a middle velocity section.

As a matter of course, the low-velocity-side velocity section of the plurality of velocity sections $S_1$ to SN may be selected as the predetermined velocity section. Alternatively, a velocity section positioned in the middle, of the plurality of velocity sections $S_1$ to SN, may be selected as the predetermined velocity section. Otherwise, the low-velocity-side velocity section and a high-velocity-side velocity section can also be selected.

The intervals (widths) of the velocity sections are also not limited, and may be arbitrarily set.

By setting the blood flow velocity section in such a manner, the heart-disease evaluation value can be computed. The heart-disease evaluation value is estimated at high accuracy by, for example, generating a learned model on the basis of information used as the input of the learning algorithm.

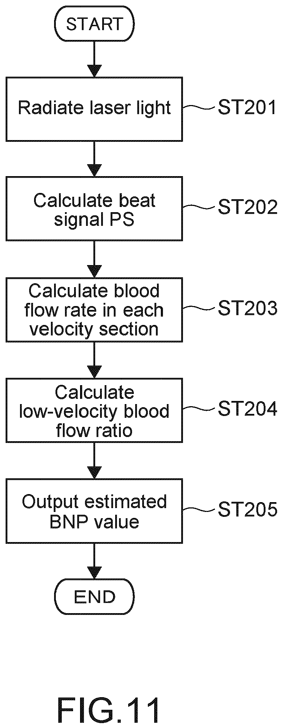

Fig. 11 is a flowchart showing a computation example of the heart-disease evaluation value based on the blood flow velocity sections. In the example shown in Fig. 11, the BNP level is estimated as the heart-disease evaluation value.

The light-emitting portion 11 of the LDF 20 radiates laser light (Step 201). Although the laser pulse light is radiated as an example, the present technology is not limited thereto.

The LDF 20 computes a beat signal PS (Step 202). The computed beat signal PS is output to the evaluation value computation device 30 as the blood flow rate information.

The evaluation value computation device 30 sets blood flow velocity sections to the beat signal PS and computes a blood flow rate F(i) in each velocity section S(i) (Step 203).

Moreover, the evaluation value computation device 30 computes a low-velocity blood flow ratio (Step 204). The low-velocity blood flow ratio corresponds to the ratio of the blood flow rate in the low-velocity-side velocity section to the blood flow rate in all the velocity sections. The low-velocity blood flow ratio is computed in accordance with the following expression, for example.

[Expression 1]

$$l = \frac{\sum_{i=0}^{M} F(i)}{\sum_{i=0}^{N} F(i)}$$

Where N represents the number of velocity sections of the plurality of velocity sections $S_1$ to SN and corresponds to a subscript N of a highest-velocity-side velocity section SN. Thus, the denominator of the expression (Formula 1) is a sum of respective blood flow rates in all the velocity sections.

M represents the number of velocity sections up to the low-velocity-side velocity section and corresponds to a subscript of an arbitrary velocity section on the lower velocity side as compared to the middle velocity section. For example, in a case where M = 1 is established, the low-velocity-side velocity section is lowest-velocity-side velocity sections $S_0$ and $S_1$. In this case, the numerator of the expression (Formula 1) is a sum of blood flow rates in the respective velocity sections $S_0$ and $S_1$.

As a matter of course, the low-velocity blood flow ratio is not limited to the low-velocity blood flow ratio defined in accordance with the expression (Formula 1).

The evaluation value computation device 30 computes a BNP level as the heart-disease evaluation value on the basis of the low-velocity blood flow ratio (Step 205). The BNP level is computed in accordance with a predetermined machine learning algorithm, for example.

It should be noted that the LDF 20 may perform Steps 203 and 204. That is, the LDF 20 may compute the blood flow rate F(i) in each velocity section S(i) or the low-velocity blood flow ratio and send it to the evaluation value computation device 30 as the blood flow rate information.

[Configuration of Heart-Disease Check Apparatus]

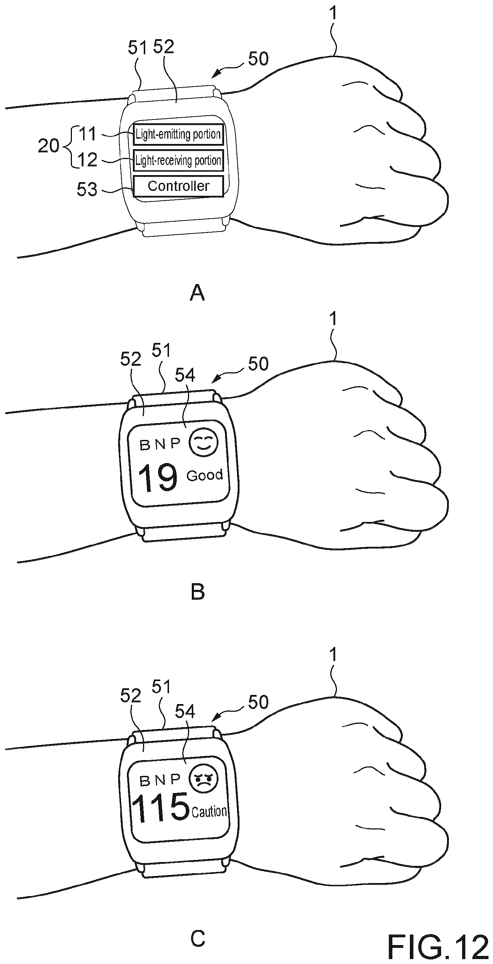

Fig. 12 is a schematic diagram showing a configuration example of a heart-disease check apparatus according to the embodiment of the present technology.

A heart-disease check apparatus 50 is a wristband-type device. The heart-disease check apparatus 50 is mounted on the wrist of the user 1 for use. That is, the heart-disease check apparatus 50 is configured as a wearable device that the user 1 can wear.

The heart-disease check apparatus 50 is an apparatus in which the sensor device 10, the evaluation value computation device 30, and the notification device 40 shown in Fig. 1 are integrally configured as a single apparatus. That is, the heart-disease check apparatus 50 includes a detection device capable of detecting a blood flow rate. In this embodiment, the LDF 20 is mounted as the detection device.

The heart-disease check apparatus 50 corresponds to an embodiment of the information processing apparatus according to the present technology. The heart-disease check apparatus 50 can also be referred to as a biometric information processing apparatus.

As shown in Fig. 12, the heart-disease check apparatus 50 includes an attachment band 51 and a sensor main body portion 52. The attachment band 51 is connected to the sensor main body portion 52, is held in contact with the wrist of the user 1, and retains the sensor main body portion 52. The specific configuration of the attachment band 51 is not limited.

As schematically shown in Fig. 12A, the sensor main body portion 52 includes the light-emitting portion 11 and the light-receiving portion 12 of the LDF 20 and a controller 53. The light-emitting portion 11 and the light-receiving portion 12 are provided on a surface side that is brought into contact with the wrist of the user 1.

As shown in Fig. 12B and C, the sensor main body portion 52 includes a display unit 54. The display unit 54 is a display device using liquid-crystal, electroluminescence (EL), or the like, for example, and is capable of displaying various images, and graphical user interfaces (GUIs), and the like. A touch panel may be configured as the display unit 54 and user's operations may be able to be input in the touch panel.

Moreover, the sensor main body portion 52 includes a speaker (not shown) capable of audio output.

The controller 53 controls operations of the respective blocks of the heart-disease check apparatus 50. The controller 53 has a hardware configuration necessary for a computer such as a CPU and a memory (RAM, ROM), for example. By the CPU loading a program stored in the ROM or the like into the RAM and executing the program, various types of processing are executed.

For example, a PLD such as an FPGA or another device such as an ASIC may be used as the controller 53.

The controller 53 has functions similar to those of the evaluation value computation device 30 shown in Fig. 1 and the signal processing device 22 shown in Fig. 6, for example. For example, the controller 53 generates blood flow rate information that is the detection result of the blood flow rate. Moreover, the controller 53 computes a heart-disease evaluation value on the basis of the blood flow rate information.

Moreover, based on the computed heart-disease evaluation value, the controller 53 generates notification information related to heart disease (video information, audio information, information for tactile presentation or light emission, or the like). The user 1 is notified of the generated notification information through the display unit 54 or the speaker.

In this embodiment, by the CPU of the controller 53 executing the program according to this embodiment, the computation unit and the generation unit are realized as the functional blocks. As a matter of course, dedicated hardware for an integrated circuit (IC) and the like may be used for realizing the functional blocks.

Moreover, in this embodiment, the display unit 54 and the speaker function as a notification unit that notifies the user 1 of the notification information related to heart disease.

It should be noted that the notification device 40 functions as the notification unit in the heart-disease check system 100 shown in Fig. 1.

In the example shown in Fig. 12B and C, the "BNP level" that is the heart-disease evaluation value, a text expressing a status of the heart disease (heart failure), e.g., "good", "caution", or the like, and a face mark image are displayed as the notification information.

The face mark image is displayed as a facial expression related to the status of the heart disease (heart failure). For example, the mark of a smile face is displayed for "good" and the mark of a facial expression feeling stressed is displayed for "caution".

Moreover, a speech saying "you are in good condition" or "you need to take care of yourself" is output as the notification information.

As a matter of course, the details of the notification information are not limited, and may be arbitrarily set.

As described above, in the heart-disease check system 100 and the heart-disease check apparatus 50 according to this embodiment, the evaluation value related to heart disease is computed on the basis of the detection result of the blood flow rate. Accordingly, the heart disease status can be easily ascertained.

The measurement of the BNP level or the NT-pro BNP level is currently widely used for diagnosis as a biomarker indicating a heart disease status. Only a medical institution can conduct the measurement because measuring those values requires a blood test.

It is important to early find a change of the heart disease status and perform suitable treatment for preventing the heart disease from becoming severe. However, it is difficult to early find deterioration of the status because frequent blood tests at a medical institution often involve pain and difficulties.

In the present technology, the BNP level or the NT-pro BNP level can be estimated non-invasively and bloodlessly without requiring a blood test at a medical institution.

Since the measurement at a medical institution becomes unnecessary, for example, remote monitoring at home or the like can be performed and a telemedicine system of heart disease can be built. For example, the computed heart-disease evaluation value is sent to a computer of the medical institution and a system that a doctor in charge or the like can check can be easily built.

For example, mounting the wristband-type heart-disease check apparatus 50 as illustrated in Fig. 12 enables the user 1 to frequently measure a BNP level or an NT-pro BNP level in blood automatically without considering the measurement in the daily life. Accordingly, a change of the heart disease status can be early found.

For example, the heart-disease check apparatus 50 is mounted and a mode to perform automatic measurement (also referred to as automatic simple diagnosis) regularly at a predetermined time every day is set. It is assumed that the status of the user 1 on a certain day is a status that there are relatively few low-velocity components of the blood flow velocity as shown in Fig. 10A. Depending on the status, the BNP level or the NT-pro BNP level is, for example, measured as a low value and the notification information as shown in Fig. 12B is notified.

Moreover, it is assumed that on another day, the status of the user 1 is a status that there are relatively many low-velocity components of the blood flow velocity as shown in Fig. 10B. Depending on the status, the BNP level or the NT-pro BNP level is, for example, measured as a high value and the notification information as shown in Fig. 12C is notified.

The user 1 checks the notification information notified regularly every day, and the heart disease status can be easily ascertained in this manner. Accordingly, the user's schedule and the like can be appropriately planned on the basis of the heart disease status, for example. Moreover, anxiety for the heart disease status can also be reduced.

<Other Embodiments>

The present technology is not limited to the above-mentioned embodiments, and various other embodiments can be realized.

In the above description, the BNP level and the NT-pro BNP level have been exemplified as the evaluation values related to heart disease. The present technology is not limited thereto, and the present technology can be applied to an arbitrary evaluation value and an arbitrary biomarker related to heart disease. In addition, the present technology can also be applied by newly setting an evaluation value related to heart disease.

In the above description, the LDF has been exemplified as the detection device capable of detecting the blood flow rate. The present technology is not limited thereto, and the present technology can also be applied to any other device such as an ultrasonic Doppler blood flowmeter.

Moreover, the present technology can also be applied to living things other than humanity, such as animals and the like.

In the above description, the wristband-type wearable device has been exemplified for the application of the present technology. The present technology is not limited thereto, and the present technology can be applied to various wearable devices. The various wearable devices include an armband-type that is attached to an upper arm, a headband-type (a head-mounted-type) that is mounted on a head, a neckband-type that is attached to a neck, a type for a body that is attached to a chest, a belt-type that is attached to a waist, an anklet-type that is attached to an ankle, a watch-type, a ring-type, a necklace-type, a clip-on earrings-type, an earrings-type, and the like.

The site at which the blood flow rate is measured is also not limited.

The application of the present technology is not limited to wearable devices that the user can wear.

For example, the present technology can be applied to a portable terminal that the user can carry, such as a smartphone, a portable phone, and a tablet terminal. That is, the information processing apparatus according to the present technology may be configured as the portable terminal.

For example, the heart-disease check apparatus according to the present technology is mounted on a smartphone or the like. When the user puts the smartphone on a site such as a wrist, the heart-disease check apparatus detects a blood flow rate and computes an evaluation value related to heart disease. Then, a touch panel or the like of the smartphone displays notification information related to heart disease. Such an embodiment can also be realized.

Moreover, the heart-disease check apparatus according to the present technology can also be mounted on an apparatus installed in a predetermined place, a movable robot, or the like. Moreover, the heart-disease check apparatus according to the present technology can also be configured in a predetermined place inside a facility or in a predetermined place inside a vehicle or the like.

For example, the heart-disease check apparatus can also be mounted on an electronic apparatus such as an audio apparatus, a TV set, and a projector, a vehicle-mounted device such as a car navigation system, an operation device such as a remote controller, various robots, an IoT device connected to the Internet or the like, and the like.

For example, the heart-disease check apparatus is mounted at a paw portion of a dog-type robot machine. The dog-type robot is made to make a move (so-called "paw" pose) of putting the robot's paw on the user's palm. At this time, the heart-disease check apparatus mounted on the paw portion of the robot measures an evaluation value related to heart disease. Such an embodiment can also be realized.

In addition, mounting the heart-disease check apparatus on a steering wheel portion of a car enables simple diagnosis to be performed on a driving user.

Using the present technology, an evaluation value of an arbitrary disease (symptom) other than the heart disease can also be computed. For example, an evaluation value related to a stroke, diabetes, or the like can also be computed on the basis of the detection result of the blood flow rate.

Fig. 13 is a block diagram showing a hardware configuration example of the evaluation value computation device 30.

The evaluation value computation device 30 includes a CPU 201, a ROM 202, a RAM 203, an input/output interface 205, and a bus 204 that connects them to one another.

A display unit 206, an input unit 207, a storage unit 208, a communication unit 209, a drive unit 210, and the like are connected to the input/output interface 205.

The display unit 206 is, for example, a display device using liquid-crystal, EL, or the like.

The input unit 207 includes, for example, a keyboard, a pointing device, a touch panel, and other operation devices. In a case where the input unit 207 includes a touch panel, the touch panel can be integral with the display unit 206.

The storage unit 208 is a nonvolatile storage device and includes, for example, an HDD, a flash memory, and other solid-state memories.

The drive unit 210 is, for example, a device capable of driving a removable recording medium 211 such as an optical recording medium and a magnetic recording tape.

The communication unit 209 includes a modem, a router, and other communication devices for communicating with other devices, which are connectable to a LAN, a WAN, and the like. The communication unit 209 may perform wired communication or may perform wireless communication. The communication unit 209 is often used separately from the evaluation value computation device 30.

Software stored in the storage unit 208, the ROM 202, or the like cooperates with hardware resources of the evaluation value computation device 30, to thereby realize information processing of the evaluation value computation device 30 having the above-mentioned hardware configuration.

Specifically, loading into the RAM 203 programs that configure the software, which are stored in the ROM 202 or the like, and executing the programs realize the information processing method according to the present technology.

The programs are, for example, installed in the evaluation value computation device 30 via the recording medium 211. Alternatively, the programs may be installed in the evaluation value computation device 30 via a global network or the like. Otherwise, an arbitrary computer-readable non-transitory storage medium may be used.

A plurality of computers communicably connected with each other via the network or the like may configure the information processing apparatus according to the present technology and may perform the information processing method and the program according to the present technology.

That is, the information processing method and the program according to the present technology may be performed not only in a computer system configured by a single computer but also in a computer system in which a plurality of computers cooperatively operate.

It should be noted that in the present disclosure, the system means a set of a plurality of components (apparatus, module (parts), and the like) and it does not matter whether or not all the components are housed in the same casing. Therefore, both of a plurality of apparatuses housed in separate casings and connected to one another via a network and a single apparatus having a plurality of modules housed in a single casing are the system.

Performing the information processing method and the program according to the present technology by the computer system includes, for example, both of a case where a single computer performs acquisition of the blood flow rate information, computation of the evaluation value related to heart disease, generation of the notification information, notification of the notification information, and the like and a case where different computers execute these processes.

Moreover, performing the respective processes by a predetermined computer includes causing another computer to some or all of those processes and acquiring the results.

That is, the information processing method and the program according to the present technology are also applicable to a cloud computing configuration in which a plurality of apparatuses shares and cooperatively processes a single function via a network.

The respective configurations such as the sensor device (detection device), the evaluation value computation device, the notification device, the LDF, and the heart-disease check apparatus, the flow of the processes such as detection of the blood flow rate, acquisition of the blood flow rate information, computation of the evaluation value related to heart disease, generation of the notification information, and notification of the notification information, and the like, which have been described with reference to the respective drawings, are merely embodiments, and can be arbitrarily modified without departing from the gist of the present technology. That is, any other configurations, algorithms, and the like for carrying out the present technology may be employed.

At least two features of the features according to the present technology which have been described above may be combined. That is, the various features described in the respective embodiments may be arbitrarily combined across the respective embodiments. Further, the above-mentioned various effects are merely exemplary and not limitative, and further other effects may be provided.

It should be noted that the present technology can also take the following configurations.

(1) An information processing apparatus, including a computation unit that computes an evaluation value related to heart disease on the basis of a detection result of a blood flow rate.

(2) The information processing apparatus according to (1), in which
the computation unit computes the evaluation value in accordance with a predetermined learning algorithm.

(3) The information processing apparatus according to (1) or (2), in which
the computation unit computes, as the evaluation value, at least one of a brain natriuretic peptide level or an N-terminal prohormone of brain natriuretic peptide level.

(4) The information processing apparatus according to any one of (1) to (3), in which
the detection result of the blood flow rate includes a detection result obtained by a laser Doppler flowmeter.

(5) The information processing apparatus according to any one of (1) to (4), in which
the computation unit divides a blood flow velocity into a plurality of velocity sections and computes the evaluation value on the basis of a blood flow rate in each velocity section.

(6) The information processing apparatus according to (5), in which
the computation unit computes the evaluation value on the basis of a ratio of the blood flow rate in each velocity section.

(7) The information processing apparatus according to any one of (1) to (6), in which
the computation unit divides a blood flow velocity into a plurality of velocity sections and computes the evaluation value on the basis of a blood flow rate in a predetermined velocity section.

(8) The information processing apparatus according to (7), in which
the computation unit computes the evaluation value on the basis of a ratio of the blood flow rate in the predetermined velocity section to a blood flow rate in all the velocity sections.

(9) The information processing apparatus according to (7) or (8), in which
the predetermined velocity section is a low-velocity-side velocity section of the plurality of velocity sections.

(10) The information processing apparatus according to any one of (1) to (9), in which
the computation unit computes the evaluation value on the basis of a time-series variation of the detection result of the blood flow rate.

(11) The information processing apparatus according to any one of (1) to (10), further including
a detection device capable of detecting the blood flow rate.

(12) The information processing apparatus according to (11), in which the detection device is a laser Doppler flowmeter.

(13) The information processing apparatus according to any one of (1) to (12), further including

a generation unit that generates notification information related to heart disease on the basis of the computed evaluation value.

(14) The information processing apparatus according to (13), further including a notification unit that notifies a user of the generated notification information.

(15) The information processing apparatus according to any one of (1) to (14), which is configured as a wearable device or a portable terminal.

(16) The information processing apparatus according to any one of (1) to (14), which is configured as a vehicle-mounted device or a part of a robot.

(17) An information processing method, including

> by a computer system,
> computing an evaluation value related to heart disease on the basis of a detection result of a blood flow rate.

(18) A program that causes a computer system to execute a step of computing an evaluation value related to heart disease on the basis of a detection result of a blood flow rate. Reference Signs List

| $S_1$ to $S_N$ | velocity section |
|---|---|
| 3 | blood vessel |
| 4 | red blood cell |
| 5 | stationary tissue |
| 10 | sensor device |
| 20 | LDF |
| 30 | evaluation value computation device |
| 40 | notification device |
| 50 | heart-disease check apparatus |
| 53 | controller |
| 100 | heart-disease check system |

**Claims**

1. An information processing apparatus, comprising a computation unit that computes an evaluation value related to heart disease on a basis of a detection result of a blood flow rate.

2. The information processing apparatus according to claim 1, wherein the computation unit computes the evaluation value in accordance with a predetermined learning algorithm.

3. The information processing apparatus according to claim 1, wherein the computation unit computes, as the evaluation value, at least one of a brain natriuretic peptide level or an N-terminal prohormone of brain natriuretic peptide level.

4. The information processing apparatus according to claim 1, wherein the detection result of the blood flow rate includes a detection result obtained by a laser Doppler flowmeter.

5. The information processing apparatus according to claim 1, wherein the computation unit divides a blood flow velocity into a plurality of velocity sections and computes the evaluation value on a basis of a blood flow rate in each velocity section.

6. The information processing apparatus according to claim 5, wherein the computation unit computes the evaluation value on a basis of a ratio of the blood flow rate in each velocity section.

7. The information processing apparatus according to claim 1, wherein the computation unit divides a blood flow velocity into a plurality of velocity sections and computes the evaluation value on a basis of a blood flow rate in a predetermined velocity section.

8. The information processing apparatus according to claim 7, wherein the computation unit computes the evaluation value on a basis of a ratio of the blood flow rate in the predetermined velocity section to a blood flow rate in all the velocity sections.

9. The information processing apparatus according to claim 7, wherein the predetermined velocity section is a low-velocity-side velocity section of the plurality of velocity sections.

10. The information processing apparatus according to claim 1, wherein the computation unit computes the evaluation value on a basis of a time-series variation of the detection result of the blood flow rate.

11. The information processing apparatus according to claim 1, further comprising a detection device capable of detecting the blood flow rate.

12. The information processing apparatus according to claim 11, wherein the detection device is a laser Doppler flowmeter.

13. The information processing apparatus according to claim 1, further comprising
a generation unit that generates notification information related to heart disease on a basis of the computed evaluation value.

**14.** The information processing apparatus according to claim 13, further comprising
a notification unit that notifies a user of the generated notification information.

**15.** The information processing apparatus according to claim 1, which is configured as
a wearable device or a portable terminal.

**16.** The information processing apparatus according to claim 1, which is configured as
a vehicle-mounted device or a part of a robot.

**17.** An information processing method, comprising

by a computer system,
computing an evaluation value related to heart disease on a basis of a detection result of a blood flow rate.

**18.** A program that causes a computer system to execute
a step of computing an evaluation value related to heart disease on a basis of a detection result of a blood flow rate.

FIG.1

FIG.2

A    Wave at frequency $f_0$

B    Wave at shifted frequency $f_0 + \Delta f$

Beat at frequency $\Delta f$

C    Interference wave

# FIG.3

# FIG.4

Light from light source

A    M$_A$

Light reflected by M$_A$

B    S$_A$1    S$_A$2

Power

f$_0$    Frequency

C    S$_A$3

Power

Beat frequency

FIG.5

FIG.6

FIG.7

Current

Time

FIG.8

FIG.9

A

B

FIG.10

START

Radiate laser light — ST201

Calculate beat
signal PS — ST202

Calculate blood
flow rate in each
velocity section — ST203

Calculate
low-velocity blood
flow ratio — ST204

Output estimated
BNP value — ST205

END

# FIG.11

51 52
50
1

20 { 11 — Light-emitting portion
12 — Light-receiving portion
53 — Controller

A

52 51
54 50
1

B N P ☺
19 Good

B

52 51
54 50
1

B N P 😠
115 Caution

C

FIG.12

FIG.13

30

201 CPU
202 ROM
203 RAM
204
205 Input/output interface
206 Display unit
207 Input unit
208 Storage unit
209 Communication unit
210 Drive unit
211 Removable recording medium

## INTERNATIONAL SEARCH REPORT

International application No.

PCT/JP2020/022939

### A. CLASSIFICATION OF SUBJECT MATTER

Int. Cl. A61B10/00(2006.01)i, A61B5/0285(2006.01)i
FI: A61B5/0285 H, A61B10/00 K

According to International Patent Classification (IPC) or to both national classification and IPC

### B. FIELDS SEARCHED

Minimum documentation searched (classification system followed by classification symbols)
Int. Cl. A61B10/00, A61B5/0285

Documentation searched other than minimum documentation to the extent that such documents are included in the fields searched
Published examined utility model applications of Japan      1922-1996
Published unexamined utility model applications of Japan    1971-2020
Registered utility model specifications of Japan            1996-2020
Published registered utility model applications of Japan    1994-2020

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | US 2018/0125376 A1 (AUBURN UNIVERSITY) 10 May 2018, paragraphs [0131]-[0133], fig. 1 | 1-2, 11, 17-18 |
| Y | | 4, 7, 10, 12-16 |
| A | | 3, 5-6, 8-9 |
| Y | JP 2015-54219 A (CASIO COMPUTER CO., LTD.) 23 March 2015, paragraphs [0002], [0011]-[0016], fig. 1, 2 | 4, 7, 12-15 |
| Y | WO 2009/081883 A1 (INSTITUTE OF NATIONAL COLLEGES OF TECHNOLOGY, JAPAN) 02 July 2009, paragraphs [0003], [0040]-[0043], fig. 8 | 10 |
| Y | WO 2018/146690 A1 (CARDIOKOL LTD.) 16 August 2018, pages 2, 32, 33 | 13-15 |

☒ Further documents are listed in the continuation of Box C.    ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 22.07.2020 | 04.08.2020 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2020/022939

C (Continuation).  DOCUMENTS CONSIDERED TO BE RELEVANT

| Category* | Citation of document, with indication, where appropriate, of the relevant passages | Relevant to claim No. |
|---|---|---|
| Y | JP 2017-124153 A (PANASONIC INTELLECTUAL PROPERTY MANAGEMENT CO., LTD.) 20 July 2017, paragraphs [0119]-[0128], fig. 13-15 | 16 |
| A | JP 2018-100968 A (KEIO UNIVERSITY) 28 June 2018, entire text, all drawings | 1-18 |
| A | JP 2017-519008 A (NOVARTIS AG) 13 July 2017, entire text, all drawings | 1-18 |

Form PCT/ISA/210 (continuation of second sheet) (January 2015)

## INTERNATIONAL SEARCH REPORT
Information on patent family members

International application No.

PCT/JP2020/022939

| Patent Documents referred to in the Report | Publication Date | Patent Family | Publication Date |
|---|---|---|---|
| US 2018/0125376 A1 | 10.05.2018 | JP 2020-501630 A paragraphs [0097]-[0099], fig. 1 CN 110198664 A | |
| JP 2015-54219 A | 23.03.2015 | (Family: none) | |
| WO 2009/081883 A1 | 02.07.2009 | US 2010/0280398 A1 paragraphs [0003], [0067]-[0072], fig. 8 | |
| WO 2018/146690 A1 | 16.08.2018 | JP 2020-507437 A paragraphs [0008]-[0010], [0210]-[0215] US 2019/0362740 A1 KR 10-2019-0113968 A CN 110494916 A | |
| JP 2017-124153 A | 20.07.2017 | US 2017/0196467 A1 paragraph [0157]-[0166], fig. 13-15 CN 106943117 A | |
| JP 2018-100968 A | 28.06.2018 | (Family: none) | |
| JP 2017-519008 A | 13.07.2017 | WO 2015/189790 A1 entire text, all drawings US 2017/0100460 A1 CN 106413740 A KR 10-2017-0018829 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017192629 A